# FASCICULE DE BREVET EUROPEEN

(11) **EP 1 889 595 B1**
(45) Date de publication et mention de la délivrance du brevet: **19.06.2019**
(21) Numéro de dépôt: 07290850.2
(22) Date de dépôt: 06.07.2007
(51) Int. Cl.: A61K 8/02, A47K 7/03

(54) **Article de nettoyage et/ou de soin de la peau et procédé de fabrication dudit article**
Gegenstand zur Reinigung und/oder Pflege der Haut und Verfahren zur Herstellung dieses Gegenstands
Cleaning and/or skin care item, and production method thereof

(30) Priorité: 04.08.2006 FR 0607134
(43) Date de publication de la demande: 20.02.2008
(73) Titulaire: Essity Operations France, 93400 Saint-Ouen (FR)
(72) Inventeur: Gregoire, Philippe, 27700 Les Andelys (FR); Bret, Bruno, 68920 Wintzenheim (FR); Louis Dit Picard, Bernard, 27370 Amfreville la Campagne (FR); Clermont, Anne-Gaëlle, 68000 Colmar (FR)
(74) Mandataire: Gevers & Orès

(56) Documents cités:
- EP-A- 1 586 308
- WO-A2-01/42548
- DE-A1- 4 000 920
- FR-A- 2 858 637
- FR-A- 2 867 067
- FR-A1- 2 781 238

## Description

L'invention concerne un article de nettoyage et/ou de soin de la peau, tel qu'un tampon à démaquiller selon le préambule de la revendication 1 et un procédé d'un tel article de nettoyage selon la revendication 17.

Dans le cadre des activités de nettoyage ou de soin de la peau, on utilise de plus en plus des supports préimprégnés, du type lingettes humides, sur lesquels des produits de nettoyage ou de soin ont été préalablement appliqués, en lieu et place du traditionnel tampon sec en coton, imprégné par la suite par l'utilisateur au moment de son usage.

Ce type de supports préimprégnés présente toutefois un certain nombre d'inconvénients.

Premièrement, du fait du caractère généralement volatile des produits appliqués, il est souvent nécessaire d'imprégner une grande quantité de produits dans les supports de manière à assurer une bonne activité nettoyante ou réparatrice desdits supports préimprégnés quelque soit le temps écoulé entre leur première exposition à l'air, en particulier lors de l'ouverture du paquet contenant lesdits supports, et leur utilisation effective. Il en résulte un coût généralement élevé pour ce type de supports.

Par ailleurs, de manière à éviter une diffusion ou une dégradation desdits produits actifs au contact de l'air, il est généralement nécessaire de prévoir des emballages totalement hermétiques pour un ou plusieurs de ces supports préimprégnés, ce qui augmente souvent de manière significative le coût de revient des articles au final et ne constitue pas toujours une solution fiable dans le temps.

Une autre solution connue consiste à ajouter aux produits actifs des conservateurs et des anti-oxydants. Cette solution s'avère également relativement onéreuse et peut selon le cas engendrer des réactions secondaires indésirables modifiant à plus ou moins long terme les propriétés et les effets des produits actifs.

Deuxièmement, ces supports préimprégnés nécessitent, pour assurer une efficacité optimale de nettoyage ou de soin et une homogénéité dans l'imprégnation, de couvrir de manière uniforme la totalité de leur surface de produits ce qui limite leur usage à une seule application possible et ne permet pas leur éventuelle réutilisation pour appliquer d'autres produits liquides en se servant de différentes zones ou faces de ces supports.

Troisièmement, du fait de la grande quantité de produits ajoutés et du caractère généralement hydrophile des supports utilisés, les produits pénètrent profondément à l'intérieur desdits supports par capillarité, engendrant souvent un transfert des produits actifs sur leur face opposée, notamment celle en contact avec la main de l'utilisateur. L'utilisation de tels supports préimprégnés s'avère donc délicate.

Par ailleurs, la diffusion des produits actifs à l'intérieur des supports risque de nuire à l'efficacité du nettoyage ou du soin. En effet, dans les conditions normales d'utilisation de tels supports préimprégnés, seuls les produits présents à la surface des supports sont en contact avec la peau et participent de ce fait à l'activité de nettoyage ou de soin.

L'invention vise donc à fournir un article de nettoyage et/ou de soin de la peau ne présentant pas les inconvénients de ce type de supports préimprégnés.

Elle vise en particulier à fournir un article de nettoyage et/ou de soin de la peau ne nécessitant pas une consommation excessive de produits de nettoyage et/ou de soin, assurant une libération mieux contrôlée desdits produits sur la peau et évitant une diffusion indésirable desdits produits à l'intérieur du support de base.

A cet effet, et conformément à l'invention, il est proposé un article de nettoyage et/ou de soin de la peau, présentant les caractéristiques de la revendication 1

Ainsi configurée, l'invention permettra donc d'éviter une consommation excessive de produits actifs et d'assurer une diffusion ou une libération graduelle desdits produits actifs sur la peau. En effet, lors du mouvement de frottement de l'article sur la peau, l'utilisateur brise progressivement les microcapsules présentes à la surface de l'article sur une zone externe spécifique et libère ainsi progressivement le contenu desdites microcapsules sur la peau.

Par ailleurs, du fait de la densité supérieure de la zone externe du support de base sur laquelle on dépose les microcapsules, laquelle se traduit par une diminution des espaces libres entre les fibres pour un volume donné, celles-ci ont moins tendance à migrer à l'intérieur du support et reste essentiellement à sa surface. Ceci évite donc de déposer des microcapsules dans le coeur du support, dans un endroit où elles risquent de ne pas se briser et où, en cas de brisure éventuelle, elles diffuseraient inutilement des produits actifs, ceux-ci n'étant pas en contact direct avec la peau.

Finalement, les produits actifs diffusés étant appliqués sur la peau juste après la brisure des microcapsules, il ne se produit pas non plus de diffusion indésirable de produits aqueux à travers le support et, donc, pas de gêne pour l'utilisateur lors de la manipulation de tels articles.
On connaît FR2867067 qui porte sur un article cosmétique à usage unique. L'article comprend un substrat insoluble imprégné d'une composition cosmétique. Le substrat est constitué de fibres hydrophiles et de fibres moins hydrophiles de manière à ce qu'une face du substrat présente une hydrophilie supérieure à l'autre. FR2781238 décrit un article textile, éventuellement de toilette, comprenant des microcapsules. Le support est un tissu qui dans les exemples est un tissu de soie de 58 g/m². Le problème du dépôt concentré sur des zones denses n'est pas évoqué. FR2858637 décrit des lingettes ou lavettes utilisées comme produits d'entretien ou cosmétique. Des microcapsules sont appliquées sur un support textile nontissé. Les exemples montrent que le support est de très faible épaisseur. EP1586308 décrit un article non tissé comprenant des microcapsules. La densification en surface n'est pas évoquée. Au contraire on y recherche une répartition uniforme des microcapsules. En outre le support est imprégné d'un liquide comme cela est indiqué dans les exemples et la revendication principale.

Les zones externes plus denses pourront se répartir de différentes manières sur le support.

En particulier, il est envisageable de prévoir plusieurs zones externes plus denses réparties de manière uniforme ou non uniforme sur un seul des côtés de l'article.

Selon une caractéristique avantageuse de l'invention, l'article comporte au moins deux zones externes plus denses disposées respectivement de chaque côté d'un support fibreux biface.

Dans une variante préférée de l'invention, les zones externes plus denses couvriront totalement et de manière uniforme les deux côtés d'un support fibreux biface.

Selon une caractéristique avantageuse de l'invention, la ou les zones externes plus denses sont constituées de fibres de coton hydrophiles.

Selon une autre caractéristique avantageuse de l'invention, la ou les zones externes plus denses sont formées par hydroliage d'un support fibreux.

Selon une autre caractéristique avantageuse de l'invention, la ou les zones externes plus denses sont formées par calandrage d'un support fibreux.

Selon une autre caractéristique de l'invention, la ou les zones externes plus denses présentent une différentiation visuelle de manière à les distinguer des autres zones externes ne comportant pas de microcapsules.

Selon une caractéristique préférée de l'invention, la ou les zones externes plus denses possèdent un motif de marquage en creux et/ou en relief.

Selon une autre caractéristique de l'invention, la ou les zones externes plus denses sont colorées ou possèdent un motif en couleur.

Selon une autre caractéristique de l'invention, les microcapsules sont colorées.

Selon une autre caractéristique avantageuse de l'invention, les microcapsules sont disposées sous forme de grappes à la surface de la ou des zones externes plus denses.

Selon une autre caractéristique de l'invention, les produits liquides contenus dans les microcapsules sont choisis parmi les vitamines, les parfums, les déodorants, les démaquillants, les hydratants, les produits solaires ou postsolaires, les anti-insectes, les anti-moustiques, les produits nettoyants pour l'hygiène des bébés, l'eau.

La liste précédente est évidemment donnée de manière indicative et non exhaustive.

Selon une autre caractéristique préférée de l'invention, les microcapsules contiennent un mélange de deux ou plusieurs produits, notamment un mélange de vitamine et de parfum.

Selon une autre caractéristique préférée de l'invention, l'article contient au moins deux types de microcapsules, notamment un premier type contenant des vitamines et un deuxième type contenant du parfum.

Selon une autre caractéristique de l'invention, les microcapsules contiennent de l'eau, laquelle est susceptible de réagir avec un ou plusieurs produits présents à l'état sec dans l'article ou ajoutés par la suite pour former une composition de nettoyage et/ou de soin de la peau.

Selon une autre caractéristique de l'invention, l'article contient au moins un premier type de microcapsules contenant un premier produit liquide et au moins un second type de microcapsules contenant un second produit liquide, lesdits premier et second produits liquides étant susceptibles de réagir entre eux pour former une composition de nettoyage et/ou de soin de la peau.

L'invention concerne également un procédé de fabrication d'un article de nettoyage et/ou de soin de la peau présentant les caractéristiques des revendications 17 à 21.

Selon une caractéristique préférée de l'invention, le dépôt des microcapsules s'opère lorsque la nappe de fibres possède une température comprise entre 10°C et 160°C et, de préférence, entre 15°C et 80°C.

Selon une autre caractéristique préférée de l'invention, les fibres artificielles ou synthétiques sont choisies notamment parmi les fibres de viscose, les fibres de polyester, les fibres bicomposantes du type polyester/polyester, polypropylène/ polypropylène, ou leurs mélanges.

Selon une autre caractéristique de l'invention, la densification de la couche externe s'opère par hydroliage.

Selon une autre caractéristique de l'invention, la densification de la couche externe s'opère par calandrage.

Ce calandrage peut se faire à froid ou à chaud, selon la nature des fibres utilisées. En particulier, le calandrage à chaud est particulièrement adapté lors d'une utilisation de fibres synthétiques thermofusibles.

La technologie mise en oeuvre pour déposer les microcapsules est choisie parmi l'impression, la pulvérisation, l'enduction, le plein bain et le foulardage.

Cette liste est évidemment donnée de manière indicative et non exhaustive.

D'autres caractéristiques et avantages de l'invention apparaîtront plus en détails dans la description qui suit.

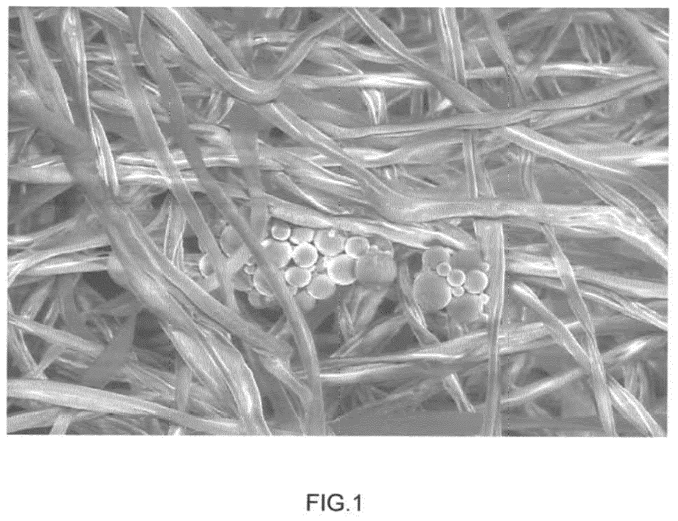

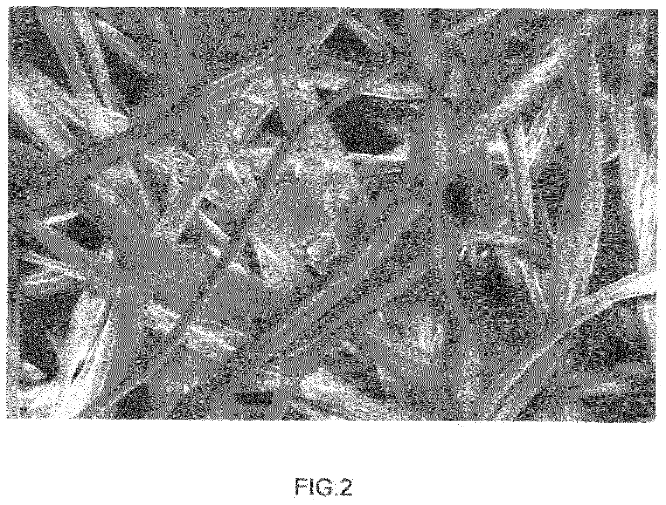

A cet égard, on fait référence aux figures 1 et 2, dans lesquelles :
- la figure 1 est une photographie prise au microscope électronique à balayage, avec un grossissement de 500 fois, de la face externe la plus hydroliée d'un tampon à démaquiller conforme à la demande de brevet internationale WO 01 425 48,
- la figure 2 est une photographie prise au microscope électronique à balayage, avec un grossissement de 800 fois, de la face externe la moins hydroliée d'un tampon à démaquiller conforme à la demande de brevet internationale WO 01 425 48.

L'encapsulation de produits liquides est connu dans les domaines cosmétique et pharmaceutique.

Cette opération consiste à protéger des microdoses de produit à l'intérieur d'une enveloppe solide en forme de capsule. La matière de l'enveloppe est choisie en fonction du produit à encapsuler et peut, par exemple, être constituée de gélatine ou collagène, d'alginates, de polysaccharides, de mélamine, de polymère amodiméthicone, de copolymère silicone ou de polyvinyle alcool.

La capsule constitue un emballage hermétique qui préserve le produit des conséquences de l'oxydation, de l'humidité, des variations de température et l'empêche de se diffuser à l'extérieur.

Elle présente généralement une taille comprise entre 1 et 3000 micromètres et, le plus souvent, comprise entre 2 et 50 micromètres.

Elle contient un taux de matières actives compris entre 20 % et 95 %, et, en général, égal à environ 40 %.

Le produit encapsulé se présente généralement sous forme d'une dispersion liquide, d'une solution liquide ou d'une émulsion stable.

Il peut également se présenter sous forme de poudre.

Il ne s'échappe des microcapsules que lors de l'éclatement de celles-ci. Il devient donc inutile de prévoir un emballage hermétique et non poreux autour des articles destinés à recevoir les microcapsules si les seuls produits à préserver sont contenus dans ces microcapsules.

L'usage de telles microcapsules est connu en particulier dans l'industrie textile, notamment pour la fabrication de sous-vêtements. Lors de l'enfilage des sous-vêtements et tout au long du port de ces derniers, les microcapsules se brisent sous l'effet de la pression, du frottement et des contraintes mécaniques diverses, libérant ainsi les produits sur la peau.

On constate toutefois que l'utilisation de telles microcapsules dans des articles de nettoyage et/ou de la soin de la peau n'a pas encore été envisagées jusqu'à présent.

Un objet de l'invention est donc d'abord de fournir des articles de nettoyage et/ou de soin de la peau possédant de telles microcapsules.

A cet effet, il est envisagé d'utiliser un support de base constitué majoritairement de fibres de coton.

De tels supports sont notamment décrits dans la demande de brevet internationale WO 01/42548 et sont commercialisés sous la marque DEMAK'UP® ou dans la demande de brevet européen EP 1 167 605 et sont commercialisés sous la marque LOTUS®.

Se présentant sous la forme de tampons de forme ronde, carrée, ovale ou rectangulaire ayant un grammage d'au moins 150 g/m², ils servent généralement au démaquillage de la peau.

Les tampons décrits dans la demande en référence possèdent par ailleurs deux faces externes différentes du point de vue de leurs caractéristiques et de leur géométrie.

En particulier, les fibres de l'une des faces externes sont plus liées que les fibres de l'autre face externe.

Ceci peut notamment résulter de l'application d'un hydroliage plus puissant sur l'une des faces externes de la nappe de coton comparativement celui appliqué sur l'autre face externe.

Ceci peut également résulter d'un marquage plus poussé sur l'une des faces externes de la nappe de coton que sur l'autre.
Il en résulte quoiqu'il en soit une densification plus grande des fibres du côté de la face la plus hydroliée ou la plus marquée.

Cette densification peut également s'opérer selon des zones localisées d'une des faces externes du tampon en coton : seules ces zones externes présenteront donc des caractéristiques et une géométrie différentes.

L'invention consiste donc à appliquer des microcapsules contenant des produits actifs de nettoyage et/ou de soin de la peau précisément sur ces zones externes plus denses de manière à favoriser au maximum le dépôt des microcapsules en surface et éviter une migration des microcapsules dans l'épaisseur du support de base.

Les figures 1 et 2 illustrent bien ce phénomène.

Ce sont des photographies prises au microscope électronique à balayage de la surface externe d'un tampon à démaquiller conforme à la demande de brevet WO 01/42548, respectivement sur sa face la plus hydroliée et sur sa face la moins hydroliée.

On a préalablement pulvérisé sur les deux faces externes de ce tampon une suspension de microcapsules selon une quantité moyenne de 1 g/m², les microcapsules possédant un diamètre inférieur à 100 micromètres, notamment inférieur à 50 micromètres et de préférence inférieur à 30 micromètres.

Le nombre de microcapsules par gramme de suspension varient entre environ 1909800 microcapsules par gramme lorsque les microcapsules ont un diamètre de 100 micromètres et environ 122230000 microcapsules par gramme lorsque les microcapsules ont un diamètre de 25 micromètres.

L'application de la suspension de microcapsules a été effectuée entre le poste de séchage et le poste de bobinage d'une machine de fabrication d'une nappe de coton destinée à former des tampons à démaquiller, telle que décrit notamment dans la demande de brevet EP 0 735 175.

Le point d'application a été notamment choisi pour éviter une détérioration des microcapsules sous l'effet de la chaleur, c'est-à-dire lorsque la température de la nappe est inférieure à la température de fusion des microcapsules.

Les microcapsules appliquées contiennent un mélange de parfum et de vitamine E.
La vitamine E est connue en cosmétique pour ses propriétés de protection de la peau vis-à-vis des radicaux libres et le vieillissement prématuré.

Le parfum sert quant à lui de révélateur olfactif de libération de la vitamine E, en complément de son pouvoir odoriférant propre.

En observant les photographies prises, on constate premièrement que les microcapsules accrochent relativement bien aux fibres de coton.

Ceci est relativement surprenant étant donné que la pulvérisation s'est opéré sans ajouter de liant de fixation.

Sans être lié par une quelconque théorie, il est envisageable de penser qu'une nappe de coton relativement sèche est apte en elle-même à retenir des microcapsules en son sein du fait de son réseau fibreux relativement dense et de ses espaces interfibres suffisamment nombreux.

On constate deuxièmement que les microcapsules s'agglutinent sous forme de grappes.

Cette agglomération des microcapsules ne se produit toutefois que pour des suspensions fortement concentrées au départ.

Dans l'exemple donné ici, la suspension possédait en l'occurrence une concentration d'environ 37 % de vitamine et de parfum par rapport au volume total de suspension.

Ceci peut être particulièrement avantageux dans le cas de l'utilisation combinée de deux types de microcapsules, l'une contenant par exemple de la vitamine et l'autre contenant du parfum.

Du fait de la présence dans une même grappe de microcapsules contenant de la vitamine et de microcapsules contenant du parfum, on libère de manière quasi systématique à la fois de la vitamine et du parfum en écrasant une de ces grappes : la détection du parfum renseigne donc l'utilisateur, de manière relativement sure, de la libération de vitamine sur la peau.

A cet égard, il est envisageable d'utiliser tout type d'actifs protecteurs de la peau à la place de la vitamine E.

Il est également envisageable d'utiliser toute autre produit liquide à l'intérieur des microcapsules, notamment des déodorants, des produits démaquillants, des produits hydratants, des produits solaires ou postsolaires, des anti-insectes, des anti-moustiques, des produits nettoyants pour l'hygiène des bébés ou tout autre produit actif cosmétique adapté.

On peut également envisager de combiner plusieurs types de produits de manière à générer un effet synergique à effet ou un avantage additionnel que ne produirait pas l'utilisation séparée de chacun des produits.

En particulier, il peut être avantageux d'appliquer sur un tampon en coton un premier type de microcapsules contenant un premier produit et un second type de microcapsules contenant un second produit, lesdits premier et second produits réagissant ensemble lorsqu'ils sont mis en contact pour former une composition de nettoyage et/ou de soin de la peau.

Ceci peut se produire notamment lorsque l'un des produits est choisi parmi les agents tensioactifs, les agents émulsionnants, les agents moussants et les gels.

Il peut également être avantageux de déposer sur un tampon en coton des microcapsules contenant de l'eau, laquelle est susceptible de réagir avec un ou plusieurs produits présents à l'état sec dans le tampon ou ajouté par l'utilisatrice de manière à former une composition de nettoyage et/ou de soin de la peau.

Finalement, on constate au vu de ses photographies une répartition des microcapsules dans les strates ou couches supérieures du tampon du côté de sa face la plus hydroliée (voir figure 1) et dans les strates ou couches sous-jacentes du tampon du côté de sa face la moins hydroliée (voir figure 2).

Il en résulte un éclatement privilégié et facilité des microcapsules présentes sur la face la plus hydroliée en cas de manipulation ou de frottement du tampon par l'utilisateur étant donné que les microcapsules disposées dans les couches internes de la nappe de fibres, du côté de la face la moins hydroliée, sont protégées par les couches supérieures.

Comme vu précédemment, ceci permet de limiter la quantité de microcapsules à appliquer sur le tampon, tout en assurant un effet de nettoyage et/ou de soin renforcé pour la peau.

En outre, afin d'optimiser au mieux cet effet, il est envisageable d'appliquer les microcapsules uniquement sur certaines zones externes de ce tampon en coton.

A cet effet, ces zones externes pourront présenter une différentiation visuelle de manière à permettre à l'utilisateur de les distinguer des autres zones externes ne comportant pas de microcapsules.

Ainsi, il est envisageable que lesdites zones externes possèdent un motif de marquage en creux et/ou en relief, un motif en couleur ou une couleur particulière, ou que les microcapsules appliquées sur lesdites zones externes soient elles-mêmes colorées.

Dans le cas du tampon en coton de l'exemple fourni, la différentiation visuelle résulte du choix d'écartement des jets utilisées pour hydrolier chacune des faces externes et de l'apport d'énergie au niveau de l'injecteur.

Il en résulte une géométrie différente pour chacune des faces externes : l'une présentant des stries en creux avec un écartement entre les stries compris entre 1 et 8 mm et une profondeur de strie d'au moins 0,25 mm et l'autre présentant des stries en creux avec un écartement entre les stries compris entre 0,4 et 1,2 mm et une profondeur de strie de l'ordre de 0,1 mm.

## Revendications

1. Article de nettoyage et/ou de soin de la peau, tel qu'un tampon à démaquiller comprenant de 70% à 100% de fibres de coton hydrophiles et de 0 à 30% de fibres artificielles ou synthétiques, et de grammage compris entre 70 et 250 g/m², **caractérisé en ce qu'**il comporte au moins une zone externe ayant une densité supérieure à celle du reste du support fibreux sur au moins une de ses faces et **en ce que** des microcapsules contenant un liquide sont disposées sur ladite au moins une zone externe ayant une densité supérieure à celle du reste du support fibreux et **en ce qu'**il ne comprend pas de liant de fixation de ces microcapsules.

2. Article selon la revendication 1, **caractérisé en ce qu'**il comporte au moins deux zones externes ayant une densité supérieure à celle du reste du support fibreux disposées respectivement de chaque côté d'un support fibreux biface.

3. Article selon l'une quelconque des revendications précédentes, **caractérisé en ce que** la ou lesdites zones externes ayant une densité supérieure à celle du reste du support fibreux sont constituées de fibres de coton hydrophiles.

4. Article selon l'une quelconque des revendications précédentes, **caractérisé en ce que** la ou lesdites zones externes ayant une densité supérieure à celle du reste du support fibreux sont formées par hydroliage du support fibreux.

5. Article selon l'une quelconque des revendications 1 à 3 précédentes, **caractérisé en ce que** la ou lesdites zones externes ayant une densité supérieure à celle du reste du support fibreux sont formées par calandrage du support fibreux.

6. Article selon l'une quelconque des revendications précédentes, **caractérisé en ce que** la ou lesdites zones externes ayant une densité supérieure à celle du reste du support fibreux présentent une différenciation visuelle de manière à les distinguer des autres zones externes ne comportant pas de microcapsules.

7. Article selon la revendication 6, **caractérisé en ce que** la ou lesdites zones externes ayant une densité supérieure à celle du reste du support fibreux possèdent un motif de marquage en creux et/ou en relief.

8. Article selon la revendication 6, **caractérisé en ce que** la ou lesdites zones externes ayant une densité supérieure à celle du reste du support fibreux sont colorées ou possèdent un motif en couleur.

9. Article selon la revendication 6, **caractérisé en ce que** les microcapsules sont colorées.

10. Article selon l'une quelconque des revendications précédentes, **caractérisé en ce que** les microcapsules sont disposées sous forme de grappes à la surface de la ou desdites zones externes ayant une densité supérieure à celle du reste du support fibreux .

11. Article selon l'une quelconque des revendications précédentes, **caractérisé en ce que** les produits liquides contenus dans les microcapsules sont choisis parmi les vitamines, les parfums, les déodorants, les démaquillants, les hydratants, les produits solaires et postsolaires, les anti-insectes, les anti-moustiques, les produits nettoyants pour l'hygiène des bébés, l'eau.

12. Article selon la revendication précédente, **caractérisé en ce que** les microcapsules contiennent un mélange d'au moins deux produits liquides.

13. Article selon la revendication 12 **caractérisé en ce que** les microcapsules contiennent un mélange de vitamine et de parfum.

14. Article selon la revendication 11, **caractérisé en ce qu'**il contient au moins deux types de microcapsules.

15. Article selon la revendication 13 **caractérisé en ce qu'**il contient un premier type de microcapsules contenant de la vitamine et un deuxième type de microcapsules contenant du parfum.

16. Article selon l'une quelconque des revendications 1 à 10, **caractérisé en ce qu'**il contient au moins un premier type de microcapsules contenant un premier produit liquide et au moins un second type de microcapsules contenant un second produit liquide, lesdits premier et second produits liquides étant susceptibles de réagir entre eux pour former une composition de nettoyage et/ou de soin de la peau.

17. Procédé de fabrication d'un article de nettoyage et/ou de soin de la peau selon l'une des revendications précédentes, comportant les étapes suivantes :
- formation d'au moins une couche externe d'un support fibreux à partir d'une nappe de fibres hydrophiles absorbantes comprenant de 70% à 100% de fibres de coton hydrophiles et de 0 à 30% de fibres artificielles ou synthétiques et de grammage compris entre 70 et 250 g/m²,
- formation d'au moins une zone de densité supérieure à celle du reste du support fibreux sur ladite couche externe,
- dépôt, sur ladite zone densité supérieure à celle du reste du support fibreux, de microcapsules contenant un ou plusieurs produits liquides susceptibles de former une composition de nettoyage et/ou de soin de la peau,
**caractérisé en ce que** le dépôt des microcapsules se fait sans ajout d'un liant de fixation.

18. Procédé selon la revendication 17, **caractérisé en ce que** le dépôt des microcapsules s'opère lorsque la nappe de fibres possède une température comprise entre 10°C et 160°C, et, de préférence, entre 15°C et 80°C.

19. Procédé selon la revendication 17 ou 18, **caractérisé en ce que** les fibres atificielles ou synthétiques sont choisies parmi les fibres de viscose, les fibres de polyester, les fibres bicomposantes du type polyester/polyester, polypropylène/polypropylène ou polyester/polypropylène ou leurs mélanges.

20. Procédé selon l'une quelconque des revendications 17 à 19, **caractérisé en ce que** la formation d'au moins une zone de densité supérieure à celle du reste du support fibreux sur la couche externe s'opère par hydroliage.

21. Procédé selon l'une quelconque des revendications 17 à 19, **caractérisé en ce que** la formation d'au moins une zone de densité supérieure à celle du reste du support fibreux sur la couche externe s'opère par calandrage.

## Patentansprüche

1. Gegenstand zur Reinigung und/oder Pflege der Haut, wie eine Watte zum Abschminken, umfassend von 70 % bis 100 % hydrophiler Baumwollfasern und von 0 bis 30 % künstlicher oder synthetischer Fasern und eine Grammatur zwischen 70 und 250 g/m², **dadurch gekennzeichnet, dass** er mindestens einen externen Bereich, der eine Dichte hat, die höher ist als diejenige des Rests des Fasersubstrats, auf mindestens einer seiner Flächen beinhaltet und dadurch, dass Mikrokapseln, die eine Flüssigkeit enthalten, auf dem mindestens einen externen Bereich angeordnet sind, der eine Dichte hat, die höher ist als diejenige des Rests des Fasersubstrats, und dadurch, dass er kein Befestigungsbindemittel dieser Mikrokapseln umfasst.

2. Gegenstand nach Anspruch 1, **dadurch gekennzeichnet, dass** er mindestens zwei externe Bereiche beinhaltet, die eine Dichte haben, die höher ist als diejenige des Rests des Fasersubstrats, die jeweils von jeder Seite eines zweiflächigen Fasersubstrats angeordnet sind.

3. Gegenstand nach einem der vorstehenden Ansprüche, **dadurch gekennzeichnet, dass** der oder die externen Bereiche, die eine Dichte haben, die höher ist als diejenige des Rests des Fasersubstrats, aus hydrophilen Baumwollfasern bestehen.

4. Gegenstand nach einem der vorstehenden Ansprüche, **dadurch gekennzeichnet, dass** der oder die externen Bereiche, die eine Dichte haben, die höher ist als diejenige des Rests des Fasersubstrats, durch Wasserstrahlbehandlung des Fasersubstrats gebildet sind.

5. Gegenstand nach einem der vorstehenden Ansprüche 1 bis 3, **dadurch gekennzeichnet, dass** der oder die externen Bereiche, die eine Dichte haben, die höher ist als diejenige des Rests des Fasersubstrats, durch Kalandern des Fasersubstrats gebildet sind.

6. Gegenstand nach einem der vorstehenden Ansprüche, **dadurch gekennzeichnet, dass** der oder die externen Bereiche, die eine Dichte haben, die höher ist als diejenige des Rests des Fasersubstrats, eine visuelle Differenzierung aufweisen, sodass sie von den anderen externen Zonen, die keine Mikrokapseln beinhalten, unterschieden werden.

7. Gegenstand nach Anspruch 6, **dadurch gekennzeichnet, dass** der oder die externen Bereiche der eine Dichte hat, die höher ist als diejenige des Rests des Fasersubstrats, ein vertieftes und/oder erhabenes Markierungsmuster besitzen.

8. Gegenstand nach Anspruch 6, **dadurch gekennzeichnet, dass** der oder die externen Bereiche, die eine Dichte haben, die höher ist als diejenige des Rests des Fasersubstrats, gefärbt sind oder ein farbiges Muster besitzen.

9. Gegenstand nach Anspruch 6, **dadurch gekennzeichnet, dass** die Mikrokapseln gefärbt sind.

10. Gegenstand nach einem der vorstehenden Ansprüche, **dadurch gekennzeichnet, dass** die Mikrokapseln in Form von Clustern an der Oberfläche des oder der externen Bereiche angeordnet sind, die eine Dichte haben, die höher ist als diejenige des Rests des Fasersubstrats.

11. Gegenstand nach einem der vorstehenden Ansprüche, **dadurch gekennzeichnet, dass** die flüssigen Produkte, die in den Mikrokapseln enthalten sind, ausgewählt sind aus Vitaminen, Parfümen, Deodorants, Abschminkmitteln, Hydratisierungsmitteln, Produkten während des Sonnenbadens und nach dem Sonnenbaden, Insektenschutzmitteln, Mückenschutzmitteln, Reinigungsprodukten für die Hygiene der Babys, Wasser.

12. Gegenstand nach dem vorstehenden Anspruch, **dadurch gekennzeichnet, dass** die Mikrokapseln ein Gemisch aus mindestens zwei flüssigen Produkten enthalten.

13. Gegenstand nach Anspruch 12, **dadurch gekennzeichnet, dass** die Mikrokapseln ein Gemisch aus Vitaminen und Parfümen enthalten.

14. Gegenstand nach Anspruch 11, **dadurch gekennzeichnet, dass** er mindestens zwei Typen von Mikrokapseln enthält.

15. Gegenstand nach Anspruch 13, **dadurch gekennzeichnet, dass** er einen ersten Typ von Mikrokapseln, die Vitamine enthalten, und einen zweiten Typ von Mikrokapseln enthält, die Parfüm enthalten.

16. Gegenstand nach einem der Ansprüche 1 bis 10, **dadurch gekennzeichnet, dass** er mindestens einen ersten Typ von Mikrokapseln, die ein erstes flüssiges Produkt enthalten, und mindestens einen zweiten Typ von Mikrokapseln enthält, die ein zweites flüssiges Produkt enthalten, wobei das erste und zweite flüssige Produkt miteinander reagieren können, um eine Zusammensetzung zur Reinigung und/oder Pflege der Haut zu bilden.

17. Herstellungsverfahren eines Gegenstands zur Reinigung und/oder Pflege der Haut nach einem der vorstehenden Ansprüche, beinhaltend die folgenden Schritte:
- Bildung mindestens einer externen Schicht eines Fasersubstrats aus einer Bahn absorbierender hydrophiler Fasern, umfassend von 70 % bis 100 % hydrophiler Baumwollfasern und von 0 bis 30 % künstlicher oder synthetischer Fasern und eine Grammatur zwischen 70 und 250 g/m²,
- Bildung mindestens eines Bereichs, dessen Dichte höher ist als diejenige des Rests des Fasersubstrats, auf der externen Schicht,
- Ablagerung auf dem Bereich, dessen Dichte höher ist als diejenige des Rests des Fasersubstrats, von Mikrokapseln, die ein oder mehrere flüssige Produkte enthalten, die eine Zusammensetzung zur Reinigung und/oder Pflege der Haut bilden können,
**dadurch gekennzeichnet, dass** die Ablagerung der Mikrokapseln ohne Zusatz eines Befestigungsbindemittels erfolgt.

18. Verfahren nach Anspruch 17, **dadurch gekennzeichnet, dass** die Ablagerung der Mikrokapseln stattfindet, wenn die Faserbahn eine Temperatur zwischen 10 °C und 160 °C und vorzugsweise zwischen 15 °C und 80 °C besitzt.

19. Verfahren nach Anspruch 17 oder 18, **dadurch gekennzeichnet, dass** die künstlichen oder synthetischen Fasern ausgewählt sind aus Viskosefasern, Polyesterfasern, Bikomponentenfasern des Typs Polyester/Polyester, Polypropylen/Polypropylen oder Polyester/Polypropylen oder Gemischen davon.

20. Verfahren nach einem der Ansprüche 17 bis 19, **dadurch gekennzeichnet, dass** die Bildung mindestens eines Bereichs, dessen Dichte höher ist als diejenige des Rests des Fasersubstrats, auf der externen Schicht durch Wasserstrahlbehandlung stattfindet.

21. Verfahren nach einem der Ansprüche 17 bis 19, **dadurch gekennzeichnet, dass** die Bildung mindestens eines Bereichs, dessen Dichte höher ist als diejenige des Rests des Fasersubstrats, auf der externen Schicht durch Kalandern stattfindet.

## Claims

1. Article for cleaning and/or for caring for the skin, such as a make-up removal pad comprising from 70% to 100% hydrophilic cotton fibres and from 0 to 30% artificial or synthetic fibres, and grammage between 70 and 250g/m², **characterised in that** it comprises at least one outer zone having a density that is greater than that of the rest of the fibrous support on at least one of the faces thereof and **in that** microcapsules containing a liquid are arranged on said at least one outer zone having a density that is greater than that of the rest of the fibrous support and **in that** it does not comprise a binder for fixation of these microcapsules.

2. Article according to claim 1, **characterised in that** it comprises at least two outer zones having a density that is greater than that of the rest of the fibrous support arranged respectively on each side of a two-face fibrous support.

3. Article according to any one of the preceding claims, **characterised in that** said outer zone(s) having a density that is greater than that of the rest of the fibrous support are formed from hydrophilic cotton fibres.

4. Article according to any one of the preceding claims, **characterised in that** said outer zone(s) having a density that is greater than that of the rest of the fibrous support are formed via spunlacing of the fibrous support.

5. Article according to any one of preceding claims 1 to 3, **characterised in that** said outer zone(s) having a density that is greater than that of the rest of the fibrous support are formed by calendering of the fibrous support.

6. Article according to any one of the preceding claims, **characterised in that** said outer zone(s) having a density that is greater than that of the rest of the fibrous support have a visual differentiation so as to distinguish them from the other outer zones that do not comprise microcapsules.

7. Article according to claim 6, **characterised in that** said outer zone or zones having a density that is greater than that of the rest of the fibrous support have a hollow and/or relief marking pattern.

8. Article according to claim 6, **characterised in that** said outer zone(s) having a density that is greater than that of the rest of the fibrous support are coloured or have a pattern in colour.

9. Article according to claim 6, **characterised in that** the microcapsules are coloured.

10. Article according to any one of the preceding claims, **characterised in that** the microcapsules are arranged in the form of clusters on the surface of said outer zone(s) having a density that is greater than that of the rest of the fibrous support.

11. Article according to any one of the preceding claims, **characterised in that** the liquid products contained in the microcapsules are selected from vitamins, perfumes, deodorants, make-up removers, moisturisers, sun protection and after-sun products, insect repellents, mosquito repellents, cleaning products for baby hygiene, water.

12. Article according to the preceding claim, **characterised in that** the microcapsules contain a mixture of at least two liquid products.

13. Article according to claim 12, **characterised in that** the microcapsules contain a vitamin and perfume mixture.

14. Article according to claim 11, **characterised in that** it contains at least two types of microcapsules.

15. Article according to claim 13, **characterised in that** it contains a first type of microcapsules containing vitamins and a second type of microcapsules containing perfume.

16. Article according to any one of claims 1 to 10, **characterised in that** it contains at least one first type of microcapsules containing a first liquid product and at least one second type of microcapsules containing a second liquid product, said first and second liquid products being likely to react together in order to form a composition for cleaning and/or for caring for the skin.

17. Method for manufacturing an article for cleaning and/or for caring for the skin according to one of the preceding claims, comprising the following steps:
- forming of at least one outer layer of a fibrous support from a layer of hydrophilic absorbent fibres comprising from 70% to 100% hydrophilic cotton fibres and from 0 to 30% artificial or synthetic fibres and grammage between 70 and 250g/m²,
- forming of at least one zone with a density greater than that of the rest of the fibrous support on said outer layer,
- depositing, on said zone with a density greater than that of the rest of the fibrous support, of microcapsules containing one or more liquid products likely to form a composition for cleaning and/or for caring for the skin,
**characterised in that** the depositing of the microcapsules is carried out without adding a binder for fixation.

18. Method according to claim 17, **characterised in that** the depositing of the microcapsules is carried out when the fibre layer has a temperature between 10°C and 160°C, and, preferably, between 15°C and 80°C.

19. Method according to claim 17 or 18, **characterised in that** the artificial or synthetic fibres are selected from viscose fibres, polyester fibres, bi-component fibres of the polyester/polyester, polypropylene/polypropylene or polyester/polypropylene type or mixtures thereof.

20. Method according to any one of claims 17 to 19, **characterised in that** the forming of at least one zone with a density greater than that of the rest of the fibrous support on the outer layer is carried out via spunlacing.

21. Method according to any one of claims 17 to 19, **characterised in that** the forming of at least one zone with a density greater than that of the rest of the fibrous support on the outer layer is carried out via calendering.
